(19) **Europäisches Patentamt**

**European Patent Office**

**Office européen des brevets**

(11) **EP 1 289 434 B1**

(12) **EUROPÄISCHE PATENTSCHRIFT**

(45) Veröffentlichungstag und Bekanntmachung des Hinweises auf die Patenterteilung:
**18.01.2006 Patentblatt 2006/03**

(21) Anmeldenummer: **01936438.9**

(22) Anmeldetag: **07.06.2001**

(51) Int Cl.:
*A61B 17/28* (2006.01)

(86) Internationale Anmeldenummer:
**PCT/EP2001/006427**

(87) Internationale Veröffentlichungsnummer:
**WO 2001/095811 (20.12.2001 Gazette 2001/51)**

(54) **MEDIZINISCHES INSTRUMENT**

MEDICAL INSTRUMENT

INSTRUMENT MEDICAL

(84) Benannte Vertragsstaaten:
**DE FR GB IT**

(30) Priorität: **10.06.2000 DE 10028896**

(43) Veröffentlichungstag der Anmeldung:
**12.03.2003 Patentblatt 2003/11**

(73) Patentinhaber: **Karl Storz GmbH & Co.**
**78532 Tuttlingen (DE)**

(72) Erfinder:
• **LANG, Dieter**
**96342 Stockheim (DE)**

• **HOPF, Thomas**
**96342 Stockheim (DE)**
• **STROBEL, Michael**
**94360 Mitterfels (DE)**

(74) Vertreter: **Hofmeister, Frank**
**Kleiststrasse 7**
**40878 Ratingen (DE)**

(56) Entgegenhaltungen:
**WO-A-98/11833**

EP 1 289 434 B1

**Beschreibung**

Die Erfindung betrifft ein medizinisches Instrument mit einem hohlen Schaft, an dessen proximalem Ende eine aus mindestens zwei Griffteilen bestehende Handhabe angeordnet ist und an dessen distalem Ende ein aus mindestens zwei Maulteilen bestehendes Werkzeug angeordnet ist, wobei mindestens ein Maulteil des Werkzeugs zum Öffnen und Schließen über jeweils ein verschwenkbar ausgebildetes Griffteil der Handhabe gegenüber dem mindestens einen anderen Maulteil des Werkzeugs verstellbar ist und das mindestens eine verstellbare Maulteil und das entsprechende, zum Verstellen des Maulteils dienende Griffteil der Handhabe über zwei Zug- /Druckstangen miteinander verbunden sind.

Gattungsgemäße Instrumente lenken das bewegliche Maulteil bzw. die beweglichen Maulteile über einen Hebelmechanismus an. Der Winkel zwischen der Instrumentenachse und der Linie durch den Drehpunkt des Maulteils und des Angriffspunkts am Hebel wird dabei möglichst groß gewählt. Beim Öffnen oder Schließen der Maulteile kommt es aber zwangsläufig zu einer Verkleinerung des Winkels, was die Kraftübertragung deutlich verschlechtert. Besonders bei medizinischen Stanzen für Gewebe ist es unbekannt, in welcher Winkelstellung der Maulteile besonders gut die Kraft übertragen werden muß, da es sich um unterschiedlich dicke Gewebe handeln kann.

Ein gattungsgemäßes medizinisches Instrument ist aus der WO 98/91833 A2 bekannt. Bei diesem bekannten, als Zange ausgebildeten medizinischen Instrument ist das verstellbare Zangenteil über zwei Zug- /Schubstangen mit dem verschwenkbaren Griffteil der Handhabe verbunden, wobei die beiden Zug-/Schubstangen beidseitig an Rollen eines Rollenlagers gelagert sind und die Rollen fest mit dem verschwenkbaren Griffteil bzw. dem verstellbaren Zangenteil verbunden sind. Die Angriffspunkte der Zug- /Schubstangen an der Rolle des verstellbaren Zangenteils sind dabei derart positioniert, daß diese oberhalb und unterhalb des Drehpunktes des verstellbaren Zangenteils auf einer Geraden liegen, die durch den Drehpunkt und diese Angriffspunkte verläuft.

Aufgrund dieses 180° Winkels, der zwischen den beiden Angriffspunkten auf dieser Geraden aufgespannt wird, ist es zwangsläufig der Fall, daß bei offener oder geschlossener Stellung des verstellbaren Zangenteils einer der für das Hebelverhältnis relevanten Winkel zwischen der Längsachse des medizinischen Instruments und einer Linie durch den Drehpunkt des verstellbaren Zangenteils und die Angriffspunkte der Zug- /Schubstangen an der Rolle deutlich über 90° beträgt. Das ideale Hebelverhältnis ergibt sich bei einem Winkel von 90°. Je größer der Winkel wird, das heißt, je weiter das Kräfteparallelogramm gestreckt wird, desto ungünstiger werden die Hebelverhältnisse zur Kraftübertragung.

Ein weiteres Instrument ist aus der US 4,712,545 A bekannt. Bei diesem bekannten chirurgischen Instrument wird das bewegliche Maulteil auf einer Bogenbahn im starren Teil des Schaftes und auf einer weiteren Bogenbahn zu einer Schubstange geführt. Diese Ausgestaltung führt zu gedachten Drehpunkten, die teilweise außerhalb des Instrumentenschafts liegen. Durch diese Maßnahme wird allerdings nur der Hebelarm vergrößert - ähnliches kann auch durch Maßnahmen an der Handhabe realisiert werden - und damit in jeder Position mehr Kraft übertragen. Je mehr aber das Kräfteparallelogramm gestreckt ist, um so ungünstiger wird das Hebelverhältnis, so daß im Extremfall auch bei großem Hebelarm keine Kraft mehr übertragen werden kann.

Ausgehend von diesem Stand der Technik liegt der Erfindung die **Aufgabe** zugrunde, ein medizinisches Instrument der eingangs genannten Art so zu verbessern, daß dieses beim Schließen der Maulteile eine immer ausreichende Krafteinleitung gewährleistet.

Die **Lösung** dieser Aufgabenstellung ist erfindungsgemäß dadurch gekennzeichnet, daß die zwei Zug- /Schubstangen an voneinander beabstandeten Angriffspunkten oberhalb und unterhalb des Drehpunktes des verstellbaren Maulteils an diesem Maulteil so festgelegt sind, daß zwei vom Drehpunkt durch je einen der Angriffspunkte ausgehende Geraden einen Winkel von 110° bis 160° aufspannen.

Durch die erfindungsgemäße Begrenzung des Winkels zwischen den beiden Angriffspunkten auf 110° bis 160° ist es möglich, die Lage der Angriffspunkte der Zug-/Schubstangen an dem verstellbaren Maulteil sowie des Drehpunktes des verstellbaren Maulteils zueinander so zu positionieren, daß die Winkel $\alpha$ und $\beta$ zwischen der Längsachse des medizinischen Instruments und einer Linie durch die Angriffspunkte der Zug- /Schubstangen am verstellbaren Maulteil sowohl in der offenen Stellung als auch in der geschlossenen Stellung jeweils etwa 90° betragen und somit ein nahezu ideales Hebelverhältnis für eine immer ausreichende Kraftübertragung gewährleisten.

Mit dieser Anordnung wird erreicht, daß beim Öffnen der Maulteile die eine Zug-/Schubstange auf Schub und die andere Stange auf Zug beansprucht wird. Beim Schließen der Maulteile kehrt sich die Arbeitsweise der Zug- /Schubstangen entsprechend um. Die Verwendung jeweils einer auf Schub und einer auf Zug beanspruchten Zug- /Schubstange erlaubt auch das Durchtrennen harten Gewebes ohne die Gefahr, daß beim Durchtrennen aufgrund des plötzlich nachlassenden Widerstands ein unkontrolliertes Durchschlagen erfolgt, da aufgrund der stets günstigen Hebelverhältnisse die Kraftübertragung jederzeit kontrollierbar ist.

Um das erfindungsgemäße medizinische Instrument gut reinigen zu können und ein einfaches und schnelles Reparieren zu ermöglichen, wird weiterhin vorgeschlagen, daß das Instrument zerlegbar ausgeführt ist.

Gemäß einer ersten Ausführungsform der Erfindung wird vorgeschlagen, daß die Zug- /Schubstangen mit dem jeweiligen Griffteil der Handhabe über eine Rastverbindung, insbesondere eine federbelastete Rastverbindung, miteinander lösbar verbunden sind. Auf diese Weise können dann die Zug-/Schubstangen gemeinsam mit der beweglichen Handhabe aus dem Schaft herausgeschwenkt werden.

Gemäß einer weiteren Ausführungsform der Erfindung wird vorgeschlagen, daß das Werkzeug über eine Bajonettkupplung am distalen Ende des Schaftes festgelegt ist und die Zug-/Schubstangen am beweglichen Griffteil lösbar sind.

Neben der Verwendung eines gemeinsamen Schaftes für alle Zug- /Schubstangen wird gemäß einer alternativen Ausführungsform vorgeschlagen, daß jede Zug-/Schubstange in einem separaten hohlen Schaft gelagert ist.

Um den Verschwenkweg der gegeneinander verschwenkbaren Griffteile der Handhabe zu verkürzen, kann die Handhabe mit einer Übersetzung versehen sein, wodurch insgesamt die Beweglichkeit des Instruments erhöht wird.

Schließlich wird mit der Erfindung vorgeschlagen, daß die mindestens zwei Maulteile des Werkzeugs über jeweils zwei Zug- /Schubstangen verstellbar ausgebildet sind, wobei jedes Maulteil über ein separates Griffteil der Handhabe betätigbar ist. Diese Ausgestaltungsform des erfindungsgemäßen medizinischen Instruments hat den Vorteil, daß die Maulteile auch in verschiedenen Winkelstellungen zum Schaft geschlossen werden können.

Weitere Merkmale und Vorteile der Erfindung ergeben sich aus der nachfolgenden Beschreibung der zugehörigen Zeichnung, in der ein Ausführungsbeispiel eines erfindungsgemäßen medizinischen Instruments beispielhaft dargestellt ist. In der Zeichnung zeigt:

Fig. 1　　eine Seitenansicht eines erfindungsgemäßen medizinischen Instruments, die Maulteile im geöffneten Zustand darstellend;

Fig. 2a　　eine vergrößerte und teilweise geschnittene Darstellung des Details IIa gemäß Fig. 1;

Fig. 2b　　eine vergrößerte und teilweise geschnittene Darstellung des Details IIb gemäß Fig. 1;

Fig. 3　　eine Seitenansicht des medizinischen Instruments gemäß Fig. 1, jedoch die Maulteile im geschlossenen Zustand darstellend;

Fig. 4a　　eine vergrößerte und teilweise geschnittene Darstellung des Details IVa gemäß Fig. 3 und

Fig. 4b　　eine vergrößerte und teilweise geschnittene Darstellung des Details IVb gemäß Fig. 3.

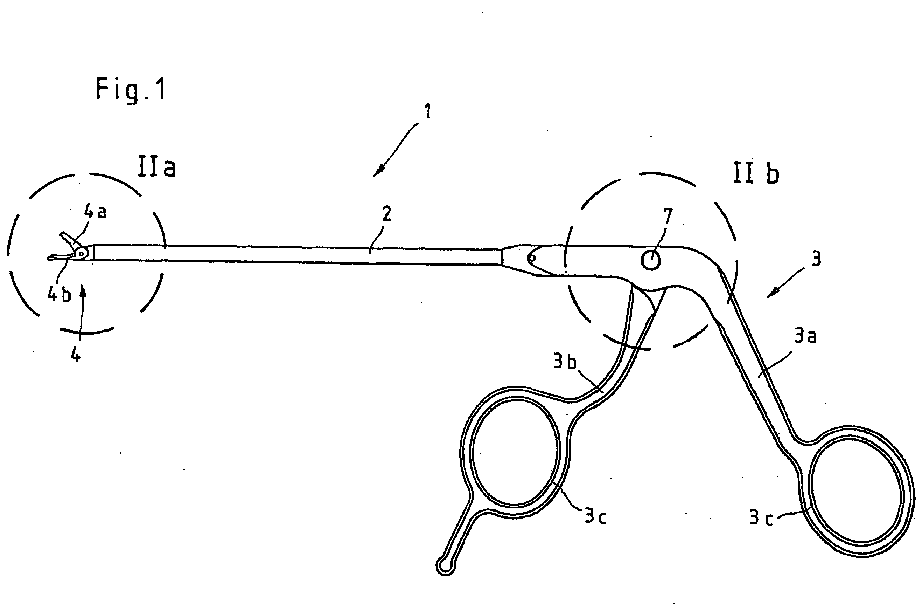

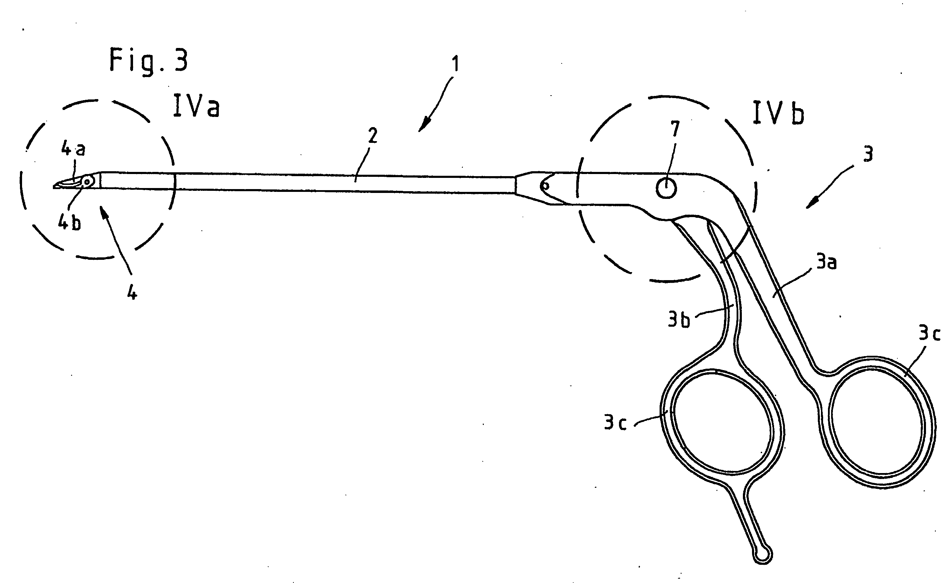

Die Abbildungen Fig. 1 und 3 zeigen Seitenansichten eines medizinischen Instruments 1, dessen Kraftübertragungsmechanismus vielseitig verwendet werden kann, wie beispielsweise für Stanzen, Scheren, Nadelhalter, Faßinstrumente und dergleichen.

Das medizinische Instrument 1 besteht im wesentlichen aus einem hohlen Schaft 2, an dessen proximalem Ende eine Handhabe 3 angeordnet ist, die aus einem starren Griffteil 3a und einem gegenüber dem starren Griffteil 3a verschwenkbaren Griffteil 3b besteht. Am distalen Ende des Schaftes 2 ist ein Werkzeug 4 angeordnet, welches beim dargestellten Ausführungsbeispiel ein verstellbares Maulteil 4a und ein starr mit dem Schaft 2 verbundenes Maulteil 4b aufweist.

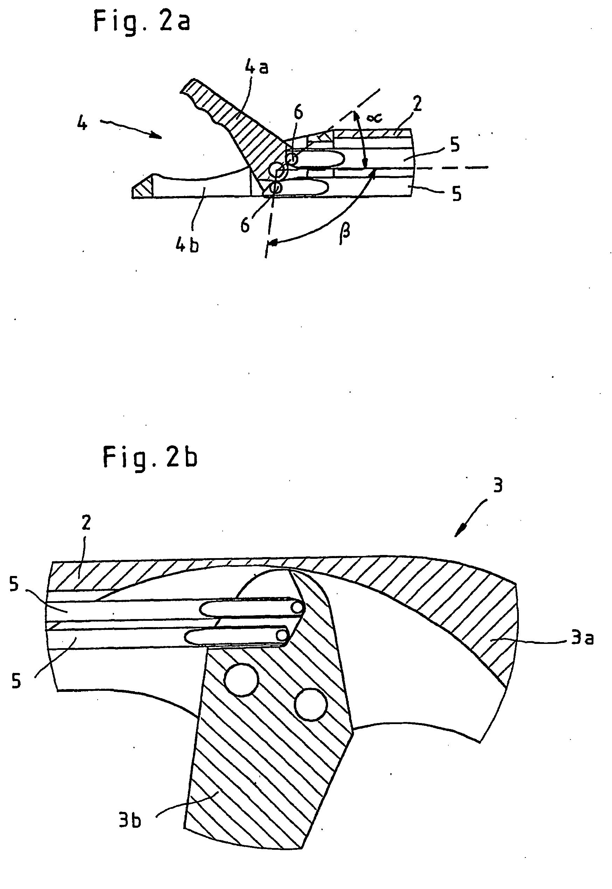

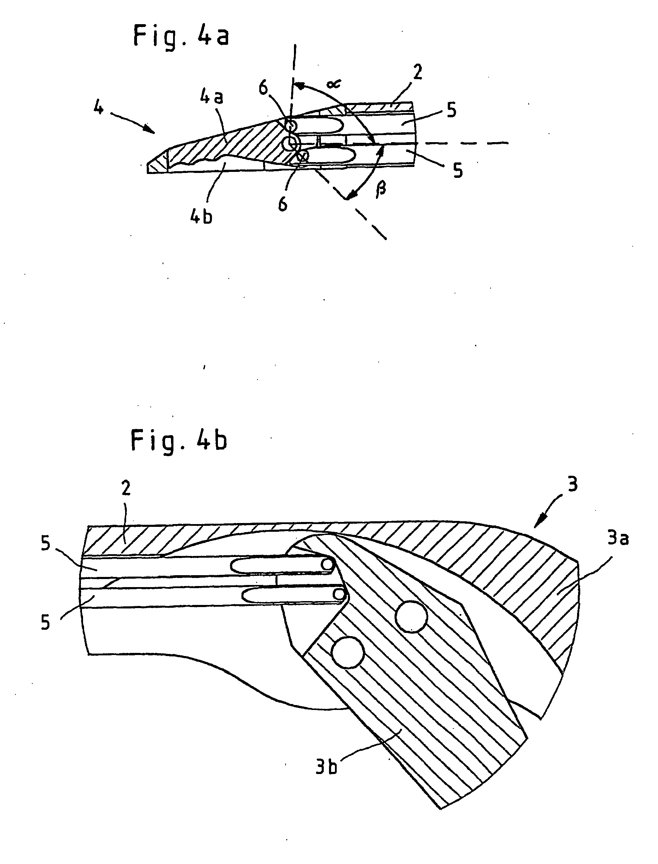

Wie aus den Detailansichten gemäß Fig. 2a, 2b und Fig. 4a, 4b ersichtlich, sind das verstellbare Maulteil 4a des Werkzeugs 4 und das verschwenkbare Griffteil 3b der Handhabe 3 über zwei Zug- /Schubstangen 5 miteinander so verbunden, daß durch das Verschwenken des Griffteils 3b das verstellbare Maulteil 4a von der geschlossenen Stellung (Fig. 3, 4a und 4b) in die offene Stellung (Fig. 1, 2a und 2b) bzw. umgekehrt überführt werden kann.

Um eine möglichst gute Kraftübertragung zwischen der Handhabe 3 und dem verstellbaren Maulteil 4a des Werkzeugs 4 zu erzielen, sind die Zug- /Schubstangen 5, wie aus Fig. 2a und 4a ersichtlich, voneinander beabstandet an Angriffspunkten 6 oberhalb und unterhalb des Drehpunktes des verstellbaren Maulteils 4a am verstellbaren Maulteil 4a so festgelegt, daß eine durch die Angriffspunkte 6 sowie den Drehpunkt gezogene Linie einen Winkel von etwa 110° bis 160° aufspannt. Durch diese Anordnung der Zug- /Schubstangen 5 wird erreicht, daß beim Öffnen des Werkzeugs 4 die obere Zug- /Schubstange 5 auf Zug beansprucht wird, während die untere Zug- /Schubstange 5 auf Schub beansprucht wird, Beim Schließen verhält es sich genau umgekehrt, das heißt, daß nun die obere Zug- /Schubstange 5 auf Schub und die untere auf Zug beansprucht wird. Die Kraftübertragung erfolgt bei dem dargestellten Ausführungsbeispiel jeweils im wesentlichen über die auf Schub beanspruchte Zug- /Schubstange 5.

Die für die Kraftübertragung günstigen Hebelverhältnisse ergeben sich auch aus dem Vergleich der Winkel, die zwischen der Längsachse des Instruments 1 und einer Linie durch die Angriffspunkte 6 der Zug- /Schubstangen 5 am Maulteil 4a und dessen Drehpunkt ausgebildet sind. Wie aus Fig. 2a ersichtlich, betragen die Winkel im offenen Zustand in etwa

$$\alpha < \beta \cong 90°.$$

Im in Fig. 4a dargestellten geschlossenen Zustand kehren sich die Verhältnisse um, so daß nunmehr gilt

$$\alpha \cong 90° > \beta,$$

so daß unabhängig vom Öffnungsgrad für die wenigstens eine Zug-/Schubstange 5 jeweils ein nahezu ideales Hebelverhältnis mit einem Winkel von etwa 90° vorliegt.

Das betätigen des medizinischen Instruments 1 geschieht wie folgt:

Zum sicheren Ergreifen der Griffteile 3a, 3b der Handhabe 3 weisen diese an ihren freien Enden Fingerösen 3c auf. Beim dargestellten Ausführungsbeispiel ist das Griffteil 3b um eine Schwenkachse 7 gegenüber dem anderen, starren Griffteil 3a verschwenkbar. Der Verschwenkweg der beiden Griffteile 3a, 3b zueinander läßt sich durch eine nicht dargestellte Übersetzung verkürzen. Ebenso ist es möglich, beide Griffteile 3a, 3b der Handhabe 3 als verschwenkbare Griffteile auszubilden.

Durch die Kopplung des verschwenkbaren Griffteils 3b über die beiden Zug-/Schubstangen 5 mit dem verstellbaren Maulteil 4a läßt sich das Werkzeug 4 öffnen und schließen. Neben der dargestellten Ausführungsform, mit nur einem verstellbaren Maulteil 4a ist es selbstverständlich auch möglich, beide Maulteile 4a, 4b verstellbar auszugestalten. In diesem Fall würden insgesamt vier Zug-/Schubstangen 5 benötigt und müßten beide Griffteile 3a, 3b der Handhabe 3 ver schwenkbar ausgestaltet sein. Vorteilhaft bei dieser nicht dargestellten Ausführungsform ist, daß die Maulteile 4a, 4b in verschiedenen Winkelstellungen zum Schaft 2 geschlossen werden können, wodurch das Instrument 1 vielseitiger verwendbar ist.

Um das Reinigen und Reparieren des medizinischen Instruments 1 zu erleichtern, können die Zug-/Schubstangen 5 lösbar mit dem Maulteil 4a bzw. dem Griffteil 3b verbunden. Hierzu eignen sich insbesondere federbelastete Rastverbindungen und Bajonettkupplungen besonders gut.

Eine weitere Möglichkeit ist die Ausführung des Drehgelenks 7 der Handhabe 3b in Form von federbelasteten Druckknöpfen, die in entsprechende Aussparungen im starren Griffteil 3a eingreifen. Nach Drücken dieser Druckknöpfe kann das verschwenkbare Griffteil 3b dann aus dem starren Griffteil 3a entnommen werden und gemeinsam mit den beiden Zug-/Schubstangen 5 nach unten aus dem dort offenen Schaft 2 ausgeschwenkt werden. Dies ermöglicht eine einfache und gründliche Reinigung bei geringem Aufwand zur Zerlegung des Instruments 1.

Insgesamt zeichnet sich das dargestellte medizinische Instrument 1 dadurch aus, daß es eine gleichmäßig starke Kraftübertragung unabhängig von der Öffnungsstellung der Maulteile gewährleistet und es möglich ist, diese Kraft dosiert einzusetzen, so daß nach dem Durchtrennen harten Gewebes kein unkontrolliertes Durchschlagen der kraftbeanspruchten Maulteile erfolgt.

## Bezugszeichenliste

| 1 | medizinisches Instrument |
|---|---|
| 2 | Schaft |
| 3 | Handhabe |
| 3a | starres Griffteil |
| 3b | verschwenkbares Griffteil |
| 3c | Fingeröse |
| 4 | Werkzeug |
| 4a | verstellbares Maulteil |
| 4b | starres Maulteil |
| 5 | Zug- /Schubstange |
| 6 | Angriffspunkt |
| 7 | Schwenkachse |
| $\alpha$ | Winkel |
| $\beta$ | Winkel |

## Patentansprüche

1. Medizinisches Instrument mit einem hohlen Schaft (2), an dessen proximalem Ende eine aus mindestens zwei Griffteilen (3a, 3b) bestehende Handhabe (3) angeordnet ist und an dessen distalem Ende ein aus mindestens zwei Maulteilen (4a, 4b) bestehendes Werkzeug (4) angeordnet ist, wobei mindestens ein Maulteil (4a) des Werkzeugs (4) zum Öffnen und Schließen über jeweils ein verschwenkbar ausgebildetes Griffteil (3b) der Handhabe (3) gegenüber dem mindestens einen anderen Maulteil (4b) des Werkzeugs (4) verstellbar ist und das mindestens eine verstellbare Maulteil (4a) und das entsprechende, zum Verstellen des Maulteils (4a) dienende Griffteil (3b) der Handhabe (3) über zwei Zug- /Druckstangen (5) miteinander verbunden sind,
**dadurch gekennzeichnet,**
**daß** die zwei Zug- /Schubstangen (5) an voneinander beabstandeten Angriffspunkten (6) oberhalb und unterhalb des Drehpunktes des verstellbaren Maulteils an diesem Maulteil (4a) so festgelegt sind, daß zwei vom Drehpunkt durch je einen der Angriffspunkte (6) ausgehende Geraden einen Winkel ($\alpha + \beta$) von 110° bis 160° aufspannen.

**2.** Medizinisches Instrument nach Anspruch 1, **dadurch gekennzeichnet, daß** die Zug- /Schubstangen (5) aus dem Schaft (2) entnehmbar sind.

**3.** Medizinisches Instrument nach Anspruch 2, **dadurch gekennzeichnet, daß** die Zug- /Schubstangen (5) zusammen mit dem jeweiligen Griffteil (3b) der Handhabe (3) über eine Rastverbindung, insbesondere eine federbelastete Rastverbindung, mit dem starren Griffteil (3a) lösbar verbunden sind.

**4.** Medizinisches Instrument nach einem der Ansprüche 1 bis 3, **dadurch gekennzeichnet, daß** das Werkzeug (4) über eine Bajonettkupplung am distalen Ende des Schaftes (2) festlegbar ist und die Zug-/Schubstangen (5) vom Griffteil (3b) lösbar sind.

**5.** Medizinisches Instrument nach einem der Ansprüche 1 bis 4, **dadurch gekennzeichnet, daß** jede Zug- /Schubstange (5) in einem separaten Schaft (2) gelagert ist.

**6.** Medizinisches Instrument nach einem der Ansprüche 1 bis 5, **dadurch gekennzeichnet, daß** zwischen den gegeneinander verschwenkbaren Griffteilen (3b) der Handhabe (3) eine Übersetzung angeordnet ist, um den Verschwenkweg der Griffteile (3a, 3b) zueinander zu verkürzen.

**7.** Medizinisches Instrument nach einem der Ansprüche 1 bis 6, **dadurch gekennzeichnet, daß** zwei Maulteile (4a, 4b) des Werkzeugs (4) verstellbar ausgebildet sind, wobei jedes Maulteil (4a, 4b) über jeweils zwei Zug-/Schubstangen (5) mit jeweils einem Griffteil (3a, 3b) der Handhabe (3) verbunden sind, die beide unabhängig voneinander verschwenkbar ausgebildet sind.

**Claims**

**1.** Medical instrument with a hollow shaft (2) which, at its proximal end, has a handle (3) consisting of at least two grip parts (3a, 3b), and, at its distal end, has a tool (4) consisting of at least two jaw parts (4a, 4b), where at least one jaw part (4a) of the tool (4) can be adjusted relative to the at least one other jaw part (4b) of the tool (4) for purposes of opening and closing via in each case a pivotable grip part (3b) of the handle (3), and the at least one adjustable jaw part (4a) and the corresponding grip part (3b) of the handle (3) that serves to adjust the jaw part (4a) are connected to one another via two pull/push rods (5), **characterized in that** the two pull/push rods (5) are secured on the jaw part (4a) at engagement points (6) spaced apart from one another above and below the pivot point of this adjustable jaw part so that two straight lines each extending from the pivot point through one of the engagement points (6) form an angle ($\alpha+\beta$) of 110° to 160°.

**2.** Medical instrument according to Claim 1, **characterized in that** the pull/push rods (5) can be removed from the shaft (2).

**3.** Medical instrument according to Claim 2, **characterized in that** the pull/push rods (5), together with the respective grip part (3b) of the handle (3), are connected releasably to the stationary grip part (3a) via a locking connection, in particular a spring-loaded locking connection.

**4.** Medical instrument according to one of Claims 1 to 3, **characterized in that** the tool (4) can be secured on the distal end of the shaft (2) via a bayonet coupling, and the pull/push rods (5) can be released from the grip part (3b).

**5.** Medical instrument according to one of Claims 1 to 4, **characterized in that** each pull/push rod (5) is mounted in a separate shaft (2).

**6.** Medical instrument according to one of Claims 1 to 5, **characterized in that**, between the grip parts (3b) of the handle (3) that are pivotable towards one another, a transmission mechanism is arranged in order to reduce the pivot path of the grip parts (3a, 3b) to one another.

**7.** Medical instrument according to one of Claims 1 to 6, **characterized in that** two jaw parts (4a, 4b) of the tool (4) are adjustable, each jaw part (4a, 4b) being connected respectively to one grip part (3a, 3b) of the handle (3) via in each case two pull/push rods (5), both of them being designed to be pivotable independently of one another.

**Revendications**

**1.** Instrument médical comprenant un corps allongé creux (2), à l'extrémité proximale duquel est disposée une poignée de manoeuvre (3) constituée d'au moins deux pièces de préhension (3a, 3b), et à l'extrémité distale duquel est disposé un outil (4) constitué d'au moins deux pièces de mâchoire (4a, 4b), au moins une pièce de mâchoire (4a) de l'outil (4) pouvant, pour l'ouverture et la fermeture, être déplacée par rapport à ladite au moins une autre pièce de mâchoire (4b) de l'outil (4), par l'intermédiaire d'une pièce de préhension respective (3b) de configuration pivotante de la poignée de manoeuvre (3), et ladite au moins une pièce de mâchoire (4a) déplaçable et la pièce de préhension (3b) correspondante de la poignée de manoeuvre (3), qui sert à déplacer la pièce de mâchoire (4a), étant reliées mutuellement par l'intermédiaire de deux tiges de traction/de compression ou de poussée,

**caractérisé en ce que** les deux tiges de traction/de poussée (5) sont fixées à la pièce de mâchoire déplaçable (4a), en des points d'action (6) espacés l'un de l'autre, au-dessus et en-dessous du point de rotation de ladite pièce de mâchoire déplaçable (4a), de façon telle que deux droites issues du point de rotation et passant chacune par l'un des points d'action (6), forment entre elles un angle $(\alpha + \beta)$ de 110° à 160°.

2. Instrument médical selon la revendication 1, **caractérisé en ce que** les tiges de traction/de poussée (5) peuvent être extraites du corps allongé (2).

3. Instrument médical selon la revendication 2, **caractérisé en ce que** les tiges de traction/de poussée (5) sont reliées de manière amovible, en commun avec la pièce de préhension (3b) respective de la poignée de manoeuvre (3), à la pièce de préhension fixe (3a), par l'intermédiaire d'une liaison par enclenchement, notamment une liaison par enclenchement chargée ou sollicitée par ressort.

4. Instrument médical selon l'une des revendications 1 à 3, **caractérisé en ce que** l'outil (4) peut être fixé par l'intermédiaire d'un accouplement du type à baïonnette, à l'extrémité distale du corps allongé (2), et les tiges de traction/de poussée (5) sont amovibles de la pièce de préhension (3b).

5. Instrument médical selon l'une des revendications 1 à 4, **caractérisé en ce que** chaque tige de traction/de poussée (5) est montée dans un corps allongé (2) séparé.

6. Instrument médical selon l'une des revendications 1 à 5, **caractérisé en ce qu'**entre les pièces de préhension (3b) de la poignée de manoeuvre (3), qui peuvent pivoter l'une par rapport à l'autre, est disposée une transmission de démultiplication, en vue de réduire le chemin de déplacement de pivotement des pièces de préhension (3a, 3b) l'une par rapport à l'autre.

7. Instrument médical selon l'une des revendications 1 à 6, **caractérisé en ce que** deux pièces de mâchoire (4a, 4b) de l'outil (4) sont d'une configuration mobile ou déplaçable, chaque pièce de mâchoire (4a, 4b) étant reliée, respectivement par l'intermédiaire de deux tiges de traction/de poussée (5), à respectivement une pièce de préhension (3a, 3b) de la poignée de manoeuvre (3), qui toutes les deux sont réalisées pivotantes indépendamment l'une de l'autre.

Fig.1

IIa

IIb

1

2

4a

4b

4

7

3

3a

3b

3c

3c

EP 1 289 434 B1

## Fig. 2a

## Fig. 2b

Fig.3

IVa

IVb

1

2

3

4a

4b

4

7

3a

3b

3c

3c

EP 1 289 434 B1

9

Fig. 4a

Fig. 4b